# EUROPEAN PATENT SPECIFICATION

(11) **EP 2 532 381 B1**
(45) Date of publication and mention of the grant of the patent: **02.09.2020**
(21) Application number: 11739368.6
(22) Date of filing: 25.01.2011
(51) Int. Cl.: A61M 25/09, A61L 31/14, A61F 2/95

(54) **MEDICAL GUIDE WIRE**
MEDIZINISCHER FÜHRUNGSDRAHT
FIL-GUIDE MÉDICAL

(30) Priority: 05.02.2010 CN 201010109134
(43) Date of publication of application: 12.12.2012
(73) Proprietor: MicroPort NeuroTech (Shanghai) Co., Ltd., Shanghai 201318 (CN)
(72) Inventor: LI, Yu, Shanghai 201203 (CN); JIN, Qiaorong, Shanghai 201203 (CN); WANG, Sen, Shanghai 201203 (CN); XIE, Zhiyong, Shanghai 201203 (CN); LUO, Qiyi, Shanghai 201203 (CN)
(74) Representative: Dantz, Jan Henning
(86) International application number: PCT/CN2011/070562
(87) International publication number: WO 2011/095089

(56) References cited:
- WO-A1-98/18516
- WO-A2-2004/011076
- CN-A- 1 897 892
- CN-A- 1 939 551
- CN-A- 101 124 008
- CN-A- 101 502 693
- CN-Y- 2 780 227
- US-A- 5 095 915
- US-A- 5 345 945
- US-A1- 2003 163 156
- US-A1- 2006 271 153
- US-A1- 2008 255 654

## Description

### Technical Field

The present application relates to the medical device field and, in particular, to a medical guide wire.

### Background Art

In an artery interventional operation, an implant instrument is generally delivered to a site of lesion of the blood vessel to reconstruct the artery wall. The delivery of the implant instrument is generally achieved by employing a guide wire in cooperation with a medical catheter. The existing guide wire generally comprises: a variable-diameter metal core, a developed and/or undeveloped coil which covers the distal end of the metal core, and a boss provided on the metal core. In delivery, the medical catheter is firstly inserted into a blood vessel which has undergone a lesion, then the implant instrument is bound to and encloses the guide wire, and the implant instrument is pushed to enter the medical catheter using the boss on the guide wire to thereby reach the site of lesion of the blood vessel.

Upon study of the prior art, the applicant finds that only the boss on the existing guide wire provides a pushing force; when the implant instrument is delivered, the bending degree of the medical catheter will change along with the circuitous blood vessel; in the position of the blood vessel where the circuiting degree is comparatively large, the resistance encountered when the implant instrument is delivered is comparatively large; and in this case, the implant instrument is likely to be kinked or deformed, which renders it impossible to complete the delivering process or adhere the implant instrument to the inner wall of the blood vessel in a pretty good manner after the delivery to influence the effect of the operation.

US 2006/271153 A1 discloses a medical guide wire with a support, which has a helical structure. It is in contact with an inner wall of a stent, for supporting this stent and maintaining the stent in a ready-to-deploy state.

US 2003/163156 A1 discloses a guidewire. A stent is arranged adjacent to a distal end of said wire between two marker bands. A sheath covers the wire.

### Summary of the Invention

In order to solve the above technical problem, the embodiments of the present application put forward a medical guide wire to achieve an integral delivery of the implant instrument into a blood vessel of the human body. The technical solutions, as claimed in claim 1 and the subsequent claims, read as follows: A medical guide wire for delivering an implant instrument, comprising: a metal core, a coil, a delivery element and a boss, wherein:
the metal core has a variable-diameter structure, which consists of a linear structure, a stepped structure and a linear structure from a proximal end to a distal end;
the number of the coil is one or more, and the coil(s) is/are fixed on the linear structure and the stepped structure at the distal end of the metal core;
the number of the delivery element is one or more, and the delivery element(s) is/are fixed on the stepped structure of the metal core;
the boss is fixed on the stepped structure of the metal core behind the delivery element, wherein the structure of the delivery element is a helical structure and wherein the delivery element is a polymer material strip film or a hollow polymer material pipe.

Preferably, the medical guide wire further comprises: a bondage element, which is provided on the metal core for binding the head end of the implant instrument.

Preferably, the medical guide wire further comprises: a protective casing, which is provided on the stepped structure of the metal core behind the boss.

Preferably, a diameter of the stepped structure gradually decreases from the proximal end to the distal end.

Preferably, the stepped structure is composed of one or more cylinders having gradually decreasing diameters.

Preferably, a middle segment portion of the metal core is composed of three cylinders having gradually decreasing diameters.

Preferably, a material of the coil is a development material through which radiation cannot pass.

Preferably, the polymer material is a thermoplastic elastomer material.

Preferably, the thermoplastic elastomer material is polyurethane, polyethylene or rubber.

Preferably, the delivery element is fixed on the metal core by thermal shrinkage, welding or glue adhering.

Preferably, a structure of the boss is an annular structure having a thickness.

Preferably, a material of the boss is a development material through which radiation cannot pass.

Preferably, the bondage element is a spring.

Preferably, the bondage element is fixed on the metal core by a nut or welding.

Preferably, the protective casing is a spring.

Preferably, the protective casing is fixed on the metal core by welding or adhering.

Preferably, the development material is gold, iridium, tungsten, platinum or tantalum.

As can be seen from the above technical solutions provided by the embodiments of the present application, the metal core of the medical guide wire is provided thereon with a plurality of delivery elements, and in delivery, the delivery element provides a pushing force in a segmented manner to make each segment of the implant instrument receive even resistance by rubbing the inner wall of the implant instrument, which prevents the implant instrument from being wrinkled or deformed due to too large resistance received by the implant instrument in delivery caused by the bending of the medical catheter, thereby integrally delivering the implant instrument into the human lumen through the medical catheter.

When the position at which the implant instrument is delivered is not ideal, a reclaim force can also be provided by the delivery element on the medical guide wire rubbing the inner wall of the implant instrument to readjust the position of the implant instrument.

Meanwhile, the medical guide wire is further provided thereon with a bondage element, which binds the head at the distal end of the implant instrument on the metal core of the medical guide wire to prevent the head end of the implant instrument from damaging and/or scratching the inner wall of the medical catheter in the delivering process and reduce the resistance received by the implant instrument in delivery, which facilitates the delivery of the implant instrument in the medical catheter,

### Brief Description of the Drawings

In order to describe the technical solutions in the embodiments of the present application or the prior art more clearly, the figures required to be used in the descriptions of the embodiments or the prior art will be briefly introduced below. Obviously, the figures in the descriptions below only are some embodiments recorded in the present application, and those skilled in the art can obtain other figures according to these figures without making inventive efforts.
Fig. 1 is a structure schematic diagram of a medical guide wire provided in Embodiment I of the present application;
Fig. 2 is a structure schematic diagram of the metal core of the medical guide wire of the embodiment of the present application; and

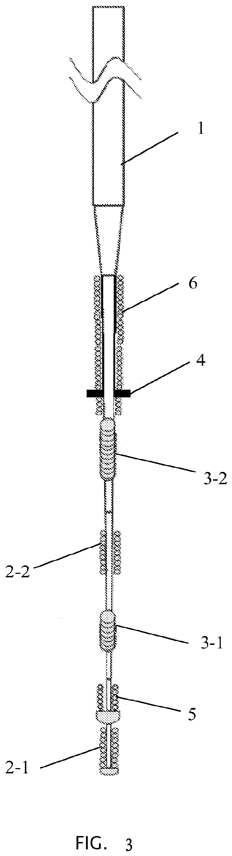

Fig. 3 is a structure schematic diagram of a medical guide wire provided in Embodiment II of the present application,

### Detailed Description

In order that those skilled in the art can better understand the technical solutions in the present application, the technical solutions in the embodiments of the present application will be clearly and completely described below in conjunction with the figures in the embodiments of the present application. Obviously, the described embodiments are only a part of the embodiments, rather than all of the embodiments, of the present application. Based on the embodiments in the present application, all the other embodiments obtained by those skilled in the art without making inventive efforts should belong to the scope of protection of the present application.

### Embodiment I:

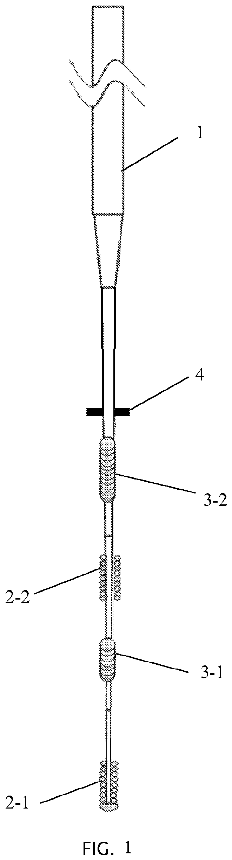

Fig. 1 is a structure schematic diagram of a medical guide wire provided in Embodiment I of the present application.

As shown in the figure, the medical guide wire comprises: a metal core 1, a coil 2, a delivery element 3 and a boss 4, wherein, the metal core 1 has a variable-diameter structure for supporting an implant instrument (not shown in the figure) in delivery; the coil 2 includes a distal portion 2-1 and a proximal portion 2-2, which are respectively fixed on the distal linear structure and the middle stepped structure of the metal core 1 for positioning the implant instrument in delivery; the delivery element 3 also includes a distal portion 3-1 and a proximal portion 3-2, which are respectively fixed on the middle stepped structure of the metal core 1 for providing a pushing force or a reclaim force for the implant instrument in delivery; and the boss 4 is an annular structure, is fixed on the middle stepped structure of the metal core 1, and is located behind the proximal portion 3-2 of the delivery element, for providing a pushing force for the implant instrument in delivery .

The embodiment of the present application has no special limitations with respect to the implant instrument delivered by the medical guide wire, and the implant instrument is preferably one well known to those skilled in the art, which includes but is not limited to a blood vessel stent, a biliary stent and an esophageal stent.

The metal core 1 is a variable-diameter spindle, which can be formed by grinding one material such as a stainless steel, a nickel-titanium alloy, a copper alloy, an aluminum alloy, etc., and can be also formed by welding or adhering several of the above materials. As shown in Fig. 1, the structure of the metal core 1 comprises a linear structure 1-3, a stepped structure 1-2 and a linear structure 1-1 from the proximal end to the distal end, wherein the diameter of the middle stepped structure 1-2 gradually decreases from the proximal end to the distal end, such a structure with its diameter gradually decreasing can be achieved by one or more segments of the stepped structure with its diameter gradually decreasing, and the middle stepped structure of the metal core 1 in the embodiment of the present application is composed of three segments.

According to the circuitous degree of the blood vessel in delivery, the metal core 1 generally decreases from the diameter of 0.012 ∼ 0.020 inch (0,3048 - 0,508 mm) of the proximal linear structure to the diameter of 0.002 ∼ 0.012 inch (0.0508 - 0,3048 mm) of the distal linear structure, the preferable range of the diameter of the proximal linear structure is 0.012 ∼ 0.016 inch (0.3048 - 0,4064 mm), and the preferable range of the diameter of the distal linear structure is 0.002 ∼ 0.005 inch (0.0508 - 0,127 mm). In addition, the length of the proximal linear structure of the metal core 1 is 1500 ∼ 2000mm, and preferably 1800 ∼ 2000mm; the length of the stepped structure is 300 ∼ 550mm, and preferably 400 ∼ 550mm; and the length of the distal linear structure is 10 ∼ 30mm. In the embodiment of the present application, the diameter of the proximal linear structure is 0.019 inch (0,4826 mm), and the length thereof is 1980mm; the length of the stepped structure is 450mm; and the diameter of the distal linear structure is 0.003 inch (0,0762 mm), and the length thereof is 15mm.

The material of the coil 2 is one or more of development materials through which radiation cannot pass such as gold, iridium, tungsten, platinum or tantalum. The distal portion 2-1 of the coil is coated on the distal linear structure of the metal core 1 for positioning the distal end of the implant instrument, i.e., in delivery, the position where the proximal end of the distal portion 2-1 of the coil is located overlaps the position where the distal end of the implant instrument is located. The proximal portion 2-2 of the coil is coated on the stepped structure of the metal core 1, the position of the proximal portion 2-2 corresponding to the central position of the delivered implant instrument, for positioning the central position of the implant instrument. The winding length of each portion of the coil 2 is 0 ∼ 30mm, and in the embodiment of the present application, the most preferable winding length of each portion of the coil 2 is 15mm.

The delivery element 3 is a polymer strip film, a metal wire or a hollow polymer pipe of a helical structure, and preferably a polymer film or polymer pipe having a high elasticity and a high friction coefficient, and the delivery element 3 is fixed to the stepped structure of the metal core 1 by thermal shrinkage, welding or glue adhering. The selected polymer material can be one or more of thermoplastic elastomers such as polyurethane, polyethylene or rubber. The coverage lengths of the distal portion 3-1 and the proximal portion 3-2 of the delivery element are 5 ∼ 8mm and 12 ∼ 15mm respectively, and the outer diameter is within the range of 0.2 ∼ 1.2mm. In delivery, the delivery element 3 binds the implant instrument on the metal core 1 by rubbing to provide the implant instrument with an even pushing force from the proximal end to the distal end, and when the position where the implant instrument is delivered is not ideal, a reclaim force can be also provided for the implant instrument in the same manner to readjust the position of the implant instrument.

The boss 4 is a ring having a wall thickness, the material of the boss 4 is a development material, and the boss 4 is used for providing a main pushing force for the implant instrument in delivery. The development design of the boss 4 is used for positioning the proximal end of the implant instrument to ensure that the implant instrument can be entirely released after being delivered.

In addition, those skilled in the art should know that the above coil and delivery element are composed of two portions, i.e., a front end portion and a proximal portion, which is only one realization mode of the present application, and in the other embodiments, the two can also be composed of a plurality of segments, which shall not constitute a limitation of the present application herein.

### Embodiment II:

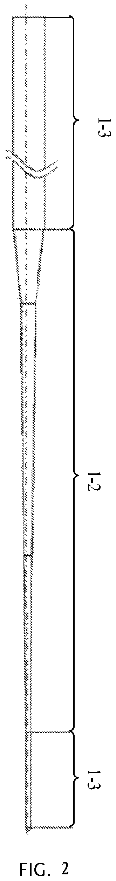

As shown in Fig. 2, the medical guide wire further comprises: a bondage element 5 and a protective casing 6, wherein the bondage element 5 is provided on the metal core 1, the position of the bondage element 5 overlapping the position where the distal end of the implant instrument is located, for binding the head end of the implant instrument; and the protective casing 5 is provided on the stepped structure of the metal core 1 behind the boss for preventing the metal core 1 from being bent.

The bondage element 5 is a spring, a material of the spring being a development material, which is fixed to the metal core 1 by a nut or welding for binding the head end of the implant instrument to prevent the head end of the implant instrument from damaging and/or scratching the inner wall of the medical catheter in the delivering process and reduce the resistance received by the implant instrument in delivery, which facilitates the delivery of the implant instrument.

In the embodiment of the present application, the metal core of the medical guide wire is provided thereon with a plurality of delivery elements, and in delivery, the delivery element provides a pushing force in a segmented manner to make each segment of the implant instrument receive even resistance by rubbing the inner wall of the implant instrument, which prevents the implant instrument from being wrinkled or deformed due to too large resistance received by the implant instrument in delivery caused by the bending of the medical catheter, thereby integrally delivering the implant instrument into the human lumen through the medical catheter.

When the position where the implant instrument is delivered is not ideal, a reclaim force can be also provided by the delivery element on the medical guide wire rubbing the inner wall of the implant instrument to readjust the position of the implant instrument,

Meanwhile, the medical guide wire is further provided thereon with a bondage element, which binds the head at the distal end of the implant instrument on the metal core of the medical guide wire to prevent the head end of the implant instrument from damaging and/or scratching the inner wall of the medical catheter in the delivering process and reduce the resistance received by the implant instrument in delivery, which facilitates the delivery of the implant instrument in the medical catheter.

## Claims

1. A medical guide wire for delivering an implant instrument, comprising: a metal core (1), a coil (2), a delivery element (3) and a boss (4), wherein:
the metal core (1) has a variable-diameter structure, which consists of a linear structure, a stepped structure and a linear structure from a proximal end to a distal end;
the number of the coil (2) is one or more, and the coil(s) (2) is/are fixed on the linear structure and the stepped structure at the distal end of the metal core (1);
the number of the delivery element (3) is one or more, and the delivery element(s) (3) is/are fixed on the stepped structure of the metal core (1); and
the boss (4) is fixed on the stepped structure of the metal core (1) proximal of the delivery element (3),
wherein the structure of the delivery element (3) is a helical structure **characterized in that** the delivery element (3) is a polymer material strip film or a hollow polymer material pipe.

2. The medical guide wire according to claim 1, **characterized by** further comprising: a bondage element (5), which is provided on the metal core (1) for binding a head end of the implant instrument.

3. The medical guide wire according to claim 1, **characterized by** further comprising: a protective casing (6), which is provided on the stepped structure of the metal core (1) proximal of the boss (4) and wherein the boss (4) is located proximal of a proximate portion of the delivery element.

4. The medical guide wire according to claim 1, **characterized in that** a diameter of the stepped structure gradually decreases from the proximal end to the distal end, preferably the stepped structure is composed of one or more cylinders having gradually decreasing diameters.

5. The medical guide wire according to claim 4, **characterized in that** a middle segment portion of the metal core (1) is composed of three cylinders having gradually decreasing diameters.

6. The medical guide wire according to one of claims 1 to 5, **characterized in that** a material of the coil (2) is a development material through which radiation cannot pass.

7. The medical guide wire according to claims 1 to 6, **characterized in that** the polymer material is a thermoplastic elastomer material.

8. The medical guide wire according to claim 7, **characterized in that** the thermoplastic elastomer material is polyurethane, polyethylene or rubber.

9. The medical guide wire according to claim 8, **characterized in that** the delivery element is fixed on the metal core (1) by thermal shrinkage, welding or glue adhering.

10. The medical guide wire according to claim 1, **characterized in that** a structure of the boss (4) is an annular structure having a thickness and that a material of the boss (4) is preferably a development material through which radiation cannot pass.

11. The medical guide wire according to claim 1, **characterized in that** the bondage element (5) is a spring which is preferably fixed on the metal core by a nut or welding.

12. The medical guide wire according to claim 1, **characterized in that** the protective casing (6) is a spring.

13. The medical guide wire according to claim 12, **characterized in that** the protective casing (6) is fixed on the metal core (1) by welding or adhering.

14. The medical guide wire according to claim 6 or 10, **characterized in that** the development material is gold, iridium, tungsten, platinum or tantalum.

## Patentansprüche

1. Medizinischer Führungsdraht zum Abgeben eines Implantatinstruments, umfassend: einen Metallkern (1), eine Spule (2), ein Abgabeelement (3) und einen Vorsprung (4), wobei:
der Metallkern (1) eine Struktur mit variablem Durchmesser aufweist, die aus einer linearen Struktur, einer stufenförmigen Struktur und einer linearen Struktur von einem proximalen Ende zu einem distalen Ende besteht;
die Anzahl der Spule (2) eins oder mehr entspricht und die Spule(n) (2) an der linearen Struktur und der stufenförmigen Struktur an dem distalen Ende des Metallkerns (1) fixiert ist/sind;
die Anzahl des Abgabeelements (3) eins oder mehr entspricht und das bzw. die Abgabeelement(e) an der stufenförmigen Struktur des Metallkerns (1) fixiert ist/sind; und
der Vorsprung (4) an der stufenförmigen Struktur des Metallkerns (1) proximal zu dem Abgabeelement (3) fixiert ist,
wobei die Struktur des Abgabeelements (3) eine schraubenförmige Struktur ist, **dadurch gekennzeichnet, dass** das Abgabeelement (3) ein Filmstreifen aus Polymermaterial oder ein Hohlrohr aus Polymermaterial ist.

2. Medizinischer Führungsdraht nach Anspruch 1, **dadurch gekennzeichnet, dass** er ferner Folgendes umfasst: ein Bindungselement (5), das an dem Metallkern (1) bereitgestellt ist, um ein Kopfende des Implantatinstruments zu verbinden.

3. Medizinischer Führungsdraht nach Anspruch 1, **dadurch gekennzeichnet, dass** er ferner Folgendes umfasst: ein Schutzgehäuse (6), das an der stufenförmigen Struktur des Metallkerns (1) proximal zum Vorsprung (4) bereitgestellt ist und wobei der Vorsprung (4) proximal zu einem darauffolgenden Abschnitt des Abgabeelements angeordnet ist.

4. Medizinischer Führungsdraht nach Anspruch 1, **dadurch gekennzeichnet, dass** ein Durchmesser der stufenförmigen Struktur von dem proximalen Ende zu dem distalen Ende allmählich abnimmt, wobei die stufenförmige Struktur vorzugsweise aus einem oder mehreren Zylindern besteht, die allmählich abnehmende Durchmesser aufweisen.

5. Medizinischer Führungsdraht nach Anspruch 4, **dadurch gekennzeichnet, dass** ein mittlerer Segmentabschnitt des Metallkerns (1) aus drei Zylindern besteht, die allmählich abnehmende Durchmesser aufweisen.

6. Medizinischer Führungsdraht nach einem der Ansprüche 1 bis 5, **dadurch gekennzeichnet, dass** ein Material der Spule (2) ein Entwicklungsmaterial ist, durch das Strahlung nicht hindurchgehen kann.

7. Medizinischer Führungsdraht nach den Ansprüchen 1 bis 6, **dadurch gekennzeichnet, dass** das Polymermaterial ein thermoplastisches Elastomermaterial ist.

8. Medizinischer Führungsdraht nach Anspruch 7, **dadurch gekennzeichnet, dass** das thermoplastische Elastomermaterial Polyurethan, Polyethylen oder Gummi ist.

9. Medizinischer Führungsdraht nach Anspruch 8, **dadurch gekennzeichnet, dass** das Abgabeelement an dem Metallkern (1) durch thermische Schrumpfung, Schweißen oder Festkleben fixiert ist.

10. Medizinischer Führungsdraht nach Anspruch 1, **dadurch gekennzeichnet, dass** eine Struktur des Vorsprungs (4) eine ringförmige Struktur ist, die eine Dicke aufweist und dadurch, dass ein Material des Vorsprungs (4) vorzugsweise ein Entwicklungsmaterial ist, durch das Strahlung nicht hindurchgehen kann.

11. Medizinischer Führungsdraht nach Anspruch 1, **dadurch gekennzeichnet, dass** das Bindungselement (5) eine Feder ist, die vorzugsweise durch eine Mutter oder Schweißen an dem Metallkern fixiert ist.

12. Medizinischer Führungsdraht nach Anspruch 1, **dadurch gekennzeichnet, dass** das Schutzgehäuse (6) eine Feder ist.

13. Medizinischer Führungsdraht nach Anspruch 12, **dadurch gekennzeichnet, dass** das Schutzgehäuse (6) an dem Metallkern (1) durch Schweißen oder Haften fixiert ist.

14. Medizinischer Führungsdraht nach Anspruch 6 oder 10, **dadurch gekennzeichnet, dass** das Entwicklungsmaterial Gold, Iridium, Wolfram, Platin oder Tantal ist.

## Revendications

1. Fil-guide médical pour la délivrance d'un instrument d'implant, comprenant : une âme en métal (1), un enroulement (2), un élément de délivrance (3) et un bossage (4), dans lequel :
l'âme en métal (1) a une structure de diamètre variable, qui est constituée d'une structure linéaire, d'une structure étagée et d'une structure linéaire d'une extrémité proximale à une extrémité distale ;
le nombre de l'enroulement (2) est d'un ou plus, et l'enroulement (les enroulements) (2) est/sont fixé(s) sur la structure linéaire et la structure étagée au niveau de l'extrémité distale de l'âme en métal (1) ;
le nombre de l'élément de délivrance (3) est d'un ou plus, et l'élément (les éléments) de délivrance (3) est/sont fixé(s) sur la structure étagée de l'âme en métal (1) ; et
le bossage (4) est fixé sur la structure étagée de l'âme en métal (1) proximale de l'élément de délivrance (3),
dans lequel la structure de l'élément de délivrance (3) est une structure hélicoïdale **caractérisé en ce que** l'élément de délivrance (3) est une bande de film en matériau polymère ou un tube de matériau polymère creux.

2. Fil-guide médical selon la revendication 1, **caractérisé en ce qu'**il comprend en outre : un élément de liaison (5), qui est prévu sur l'âme en métal (1) pour lier une extrémité de tête de l'instrument d'implant.

3. Fil-guide médical selon la revendication 1, **caractérisé en ce qu'**il comprend en outre : une enveloppe protectrice (6), qui est prévue sur la structure étagée de l'âme en métal (1) proximale du bossage (4) et dans lequel le bossage (4) est situé proximal d'une portion proche de l'élément de délivrance.

4. Fil-guide médical selon la revendication 1, **caractérisé en ce qu'**un diamètre de la structure étagée décroît progressivement de l'extrémité proximale à l'extrémité distale, de préférence la structure étagée est composée d'un ou de plusieurs cylindres dont les diamètres sont progressivement décroissants.

5. Fil-guide médical selon la revendication 4, **caractérisé en ce qu'**une portion de segment de milieu de l'âme en métal (1) est composée de trois cylindres ayant des diamètres progressivement décroissants.

6. Fil-guide médical selon l'une des revendications 1 à 5, **caractérisé en ce qu'**un matériau de l'enroulement (2) est un matériau de développement à travers lequel un rayonnement ne peut pas passer.

7. Fil-guide médical selon les revendications 1 à 6, **caractérisé en ce que** le matériau de polymère est un matériau élastomère thermoplastique.

8. Fil-guide médical selon la revendication 7, **caractérisé en ce que** le matériau élastomère thermoplastique est le polyuréthane, le polyéthylène ou le caoutchouc.

9. Fil-guide médical selon la revendication 8, **caractérisé en ce que** l'élément de délivrance est fixé sur l'âme en métal (1) par retrait thermique, soudage ou adhérence à la colle.

10. Fil-guide médical selon la revendication 1, **caractérisé en ce qu'**une structure du bossage (4) est une structure annulaire ayant une épaisseur et **en ce qu'**un matériau du bossage (4) est de préférence un matériau de développement à travers lequel un rayonnement ne peut pas passer.

11. Fil-guide médical selon la revendication 1, **caractérisé en ce que** l'élément de liaison (5) est un ressort qui est de préférence fixé sur l'âme en métal par un écrou ou soudage.

12. Fil-guide médical selon la revendication 1, **caractérisé en ce que** l'enveloppe protectrice (6) est un ressort.

13. Fil-guide médical selon la revendication 12, **caractérisé en ce que** l'enveloppe protectrice (6) est fixée sur l'âme en métal (1) par soudage ou adhérence.

14. Fil-guide médical selon la revendication 6 ou 10, **caractérisé en ce que** le matériau de développement est l'or, l'iridium, le tungstène, le platine ou le tantale.
